Europäisches Patentamt

European Patent Office

Office européen des brevets

(11) Publication number: **0 165 523**
**B1**

(12) **EUROPEAN PATENT SPECIFICATION**

(45) Date of publication of patent specification: **24.05.89**

(21) Application number: **85106792.6**

(22) Date of filing: **04.03.82**

(60) Publication number of the earlier application in accordance with Art. 76 EPC: **0 061 843**

(51) Int. Cl.⁴: **A 61 B 5/00**

(54) **Apparatus for measuring brain activity.**

(30) Priority: **28.03.81 JP 44179/81**
**30.07.81 JP 118455/81**
**06.08.81 JP 122393/81**
**06.08.81 JP 122394/81**
**06.08.81 JP 122395/81**
**06.08.81 JP 122396/81**

(43) Date of publication of application:
**27.12.85 Bulletin 85/52**

(45) Publication of the grant of the patent:
**24.05.89 Bulletin 89/21**

(84) Designated Contracting States:
**DE FR GB IT NL**

(56) References cited:
**US-A-3 858 574**
**US-A-4 183 360**

(73) Proprietor: **Nakamatsu, Yoshiro**
**1-10-309, 5-chome, Minami Aoyama Minato-ku**
**Tokyo (JP)**

(72) Inventor: **Nakamatsu, Yoshiro**
**1-10-309, 5-chome, Minami Aoyama Minato-ku**
**Tokyo (JP)**

(74) Representative: **Shaw, Laurence**
**George House George Road**
**Edgbaston Birmingham B15 1PG (GB)**

EP 0 165 523 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

It is known from U.S. Patent—A—4183360 to use a photo optical instrument to detect and record the rate of blood flow through different fingers. The system comprises means for receiving part of the body of a person, means for radiating light through the body part and means for receiving the light transmitted through the body part and converting it into an electrical signal which is then recorded on a chart.

It is one object of this invention to provide the measurement of the mental activity of the brain of a person, by measuring a physical change taking place within the brain as a result of the mental activity. This invention is based on the use of apparatus for measuring blood flow and detecting a change in blood flow to indicate a change in the level of mental activity.

The invention provides use of apparatus comprising a socket (463) for receiving a part (464) of the body of a person, means (468) for radiating light through the body part (464), means (470) for receiving the light transmitted through the body part (464) and converting it into an electrical signal and meter means (447, 448) for measuring the size of the signal characterised in that the apparatus is used to detect brain activity by taking a change in flow of blood in the body part (464) as a measure of change in brain activity.

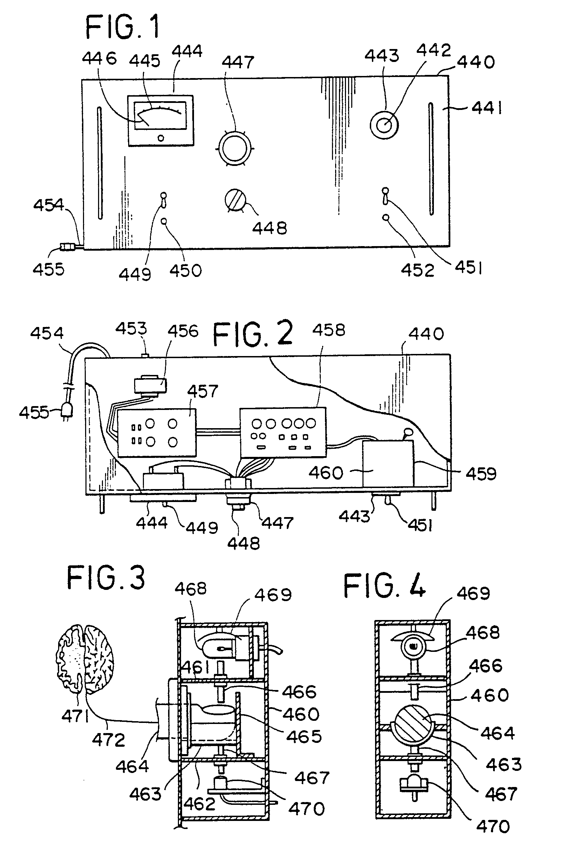

In order that the invention may be well understood it will now be described with reference to the accompanying drawings, in which Figure 1 is a front elevation of an apparatus useful for measuring brain power and Figure 2 is a top view, partly in section, thereof;

Figures 3 and 4 are sectional views showing detail of the apparatus of Figures 1 and 2; and

Figure 5 is a circuit diagram incorporating the apparatus of Figure 1;

The apparatus of Figures 1 to 5 is useful in measuring the brain activity of a person. The measuring apparatus comprises a housing 440 having a front panel 441. The front panel 441 includes a finger-socket 442 having a protective gasket 443. The front panel 441 includes a meter 444 having a scale 445 with a needle 446, a tuning knob 447 for roughly adjusting the needle 446 on the scale 445, a fine tubing knob 448, a power switch 449, a display lamp 450 indication that power is on, a switch 451 for controlling a source of light and a display lamp 452 for indicating light-on. Within the housing 440 are mounted a fuse 453 for the power source, a power line 454 and an attaching plug 455, a power transformer 456, a rectifier 457, an amplifier 458 and a measuring section 459. The measuring section 459 includes a housing 460 located behind the finger-socket 442 of the panel 441. Referring to Figures 3 and 4, the housing 460 includes upper and lower bridging bars 461 and 462. The lower bar 462 supports a trough-like finger socket 463 for a human finger 464. The lower bar 462 also supports a stopper 465 for positioning the finger 464 at a predetermined location in the finger receiver 463. The upper bar 461 supports a vertical transparent acrylic rod or optical fiber 466 and the lower bar 462 supports another such another such transparent rod 467 in vertical alignment with the upper rod 466. A lamp light source 468 is mounted in the housing 460 above the upper transparent rod 466, and has a top reflector 469. A photoreceptor 470 is mounted near the lower transparent rod 467.

When the finger 464 is received in the finger receiver 463 and the lamp 468 is turned on, light is transmitted along the upper transparent rod 466, through the positioned finger 464 and along the lower transparent rod 467 to the photoreceptor 470. The blood vessels in the finger 464 are connected to the brain 471 through vessels 472; if the flow of blood in the brain 471 is changed the flow of blood in the finger 464 is correspondingly changed and in this way a change in brain activity can be detected by measuring the flow of blood in the finger 464 which will appear at the photoreceptor 470 as any change of photocurrent.

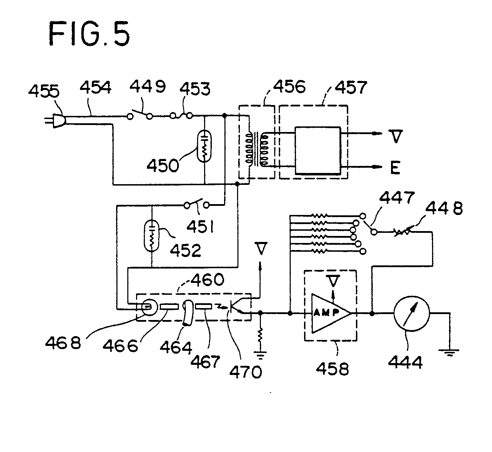

In use, the apparatus works as shown in the circuit diagram of Figure 5. The power switch 449 is switched on, the finger 464 is inserted into the receiver 463 through the socket 442 until it engages the stopper 465. The light switch 451 is turned on and then the adjusting knobs 447 and 448 are moved to obtain zero or like base indication of the indicator 446 in the meter 444. The finger 464 is then rotated around its central axis until the needle 446 shows the minimum value. If the needle 446 is moved beyond the maximum value of the scale 445 the needle 446 is brought within the scale 445 by adjusting the knobs 447 and 448. The light switch 451 is turned off and the finger 464 is removed from the opening 442. The finger 464 is again inserted into the finger receiver 463 and the light switch 451 is turned on to measure a measurement in the meter 444 without movement of the adjusting knobs 447 and 448. This measurement is compared with the previous measurement to measure an increase of brain power.

## Claim

Use of apparatus comprising a socket (463) for receiving a part (464) of the body of a person, means (468) for radiating light through the body part (464), means (470) for receiving the light transmitted through the body part (464) and converting it into an electrical signal and meter means (447, 448) for measuring the size of the signal characterized in that the apparatus is used to detect brain activity by taking a change in the flow of blood in the body part (464) as a measure of a change in brain activity.

## Patentanspruch

Einsatz eines Apparates bestehend aus einer Hülse (463) zur Aufnahme eines menschlichen Körperteils (464), Einrichtung (468) zur Durchstrahlung des Körperteils (464), Einrichtung (470)

zum Empfang und zur Umwandlung in elektrische Signale des durch den Körperteil (464) gestrahlten Lichtes sowie Meßeinrichtungen (447, 448) zur Messung des Signals, dadurch gekennzeichnet, daß der Apparatus dafür eingesetzt wird, Gehirnfunktionen zu messen, indem die Blutströmung im Körperteil (464) als Maß für einen Wechsel der Gehirnfunktionen herangezogen wird.

**Revendication**

Mode de l'amploi de l'appareil qui comprend une cavité (463) destinée à recevoir une partie quelconque du corps humain (464), les moyens (468) de diffuser la lumière à travers cette partie du corps (464), les moyens (470) de recevoir la lumière transmise au travers de cette partie du corps (464) et de la convertir en signal électrique et moyen de mensuration (447, 448) servant à calculer la dimension du signal, caractérisé en ce que l'appareil est employé pour déceler l'activité du cerveau en prenant un changement de la circulation du sang de la partie du corps (464) comme mesure du changement de l'activité cérébrale.

FIG.1

FIG.2

FIG.3

FIG.4

# FIG.5